# EUROPEAN PATENT APPLICATION

(11) **EP 4 421 086 A1**
(43) Date of publication of application: **28.08.2024**
(21) Application number: 23382162.8
(22) Date of filing: 23.02.2023
(51) Int. Cl.: C07K 14/47, C12N 9/00, A61K 48/00, C12N 15/86

(54) **NUCLEIC ACID VECTOR COMPRISING A CODING SEQUENCE OF THE EPM2A GENE**

(71) Applicant: Fundacion Instituto De Investigacion Sanitaria Fundacion Jimenez Diaz, 28040 Madrid (ES)
(72) Inventor: SERRATOSA FERNÁNDEZ, José María, 28040 Madrid (ES); SÁNCHEZ GARCÍA, Marina, 28040 Madrid (ES); ZAFRA PUERTA, Luis, 28040 Madrid (ES)
(74) Representative: Hoffmann Eitle

(57) **Abstract**

The present invention refers to a nucleic acid vector to be used in the context of gene therapy in the treatment of Lafora disease.

## Description

### FIELD OF THE INVENTION

The present invention refers to the medical field. Particularly, it refers to a nucleic acid vector to be used in the context of gene therapy in the treatment of Lafora disease.

### STATE OF THE ART

Lafora disease (OMIM #254780; ORPHA: 501) is a rare form of progressive myoclonic epilepsy that usually appears early in adolescence. The disease was first described by Spanish neurologist Gonzalo Rodriguez-Lafora in 1911. The main symptoms are seizures and a general neurological deterioration with dementia that leads to death, usually 5 to 15 years after disease onset. There is no specific therapy and patients can only be treated with antiseizure medications to temporarily control epileptic seizures. Lafora disease is caused by autosomal recessive mutations in two genes: EPM2A, encoding for a dual-specificity phosphatase called laforin, or EPM2B, encoding for malin, an E3-ubiquitin ligase. Laforin and malin form a complex that regulates glycogen synthesis by inducing the proteasome-dependent degradation of muscle glycogen synthase (MGS) and protein targeting to glycogen (PTG). Alterations in laforin or malin cause problems in the complex formation, leading to the appearance of periodic-acid-Schiff (PAS) positive inclusions known as Lafora bodies (LBs), composed of an aberrant type of insoluble, poorly branched, and hyperphosphorylated glycogen.

Two murine models of Lafora disease with targeted deletions of the Epm2a-/- or Epm2b-/- genes have been generated. Both models show some of the main neurological alterations that occur in patients. Two neuroprotective drugs have been tested in these mice, 4-phenylbutyric acid (4-PBA), which regulates proteostasis, and metformin, an AMPK activator. Metformin has shown very exciting results both in animal models and in patients. The positive results derived from experiments in animal models of Lafora disease resulted in the designation of metformin as an orphan drug for the treatment of Lafora disease by the European Medicines Agency (EMA) in 2016 and by the United States Food and Drug Administration (FDA) in 2017.

In the last years, the relevance of gene therapy for the treatment of rare diseases has increased. Recombinant adeno-associated viruses (rAAVs) have emerged as one of the most important vectors for gene therapy, as they rarely integrate into the host genome, produce low immunogenicity, are non-pathogenic, have broad tropism, and allow long-term expression of the transgene. These viruses belong to the Parvoviridae family, genus Dependoviridae, and were first isolated in the 1960s. They are small non-enveloped single-stranded (ss) DNA particles. So far, different rAAV serotypes and more than 100 variants have been isolated from adenovirus stocks or from human/non-human primate tissues. All share similar structure, DNA size and organization. The DNA genome of AAVs has a size of ~4,7 kb and includes two inverted terminal repeat (ITR) regions flanking two large open reading frames (ORFs). The ITRs are the cis-acting elements required for genome replication and packaging. The replication (rep) genes, which form the left ORF, encode four proteins (Rep78, Rep68, Rep 52 y Rep40) needed for site-specific integration, nicking, helicase activity, and regulation of promoters within the AAV genome. The capsid (cap) genes, which form the right ORF, encode three structural proteins (VP1, VP2 and VP3) that assemble into icosahedral virion shells. This icosahedral capsid contains 60 copies of the three structural proteins in a 1:1:18 ratio (VP1, VP2 and VP3, respectively). Different serotypes have different tropism and recent progress in the field allow for improvement and transfection in the tissue of interest.

Voretigene neparvovec-rzyl (Luxturna^{®}) is an AAV serotype 2 containing RPE65 that, after promising results in animal models and clinical trials, was designated as an orphan drug by the EMA to treat retinitis pigmentosa in 2015 and Leber congenital amaurosis in 2012. Its use as a therapy to treat inherited retinal dystrophy caused by mutations in RPE65 was approved in 2017 by the FDA and in 2018 by the EMA. Onasemnogene abeparvovec (Zolgensma^{®}) is an AAV serotype 9 containing the human spinal muscular atrophy (SMN) gene. After promising results, Zolgensma^{®} was approved for medical use in 2019 by the FDA and in 2020 by the EMA to treat SMA type 1. According to clinicaltrials.gov, two clinical trials are in process to treat epilepsy. After showing efficacy in refractory focal epilepsy in animal models, a lentiviral-based gene therapy is being tested in a phase II clinical trial to introduce an engineered potassium channel into the area of the brain where seizures arise. ETX101, a recombinant adeno-associated virus serotype 9 that encapsulates a GABAergic regulatory element (reGABA) and an engineered transcription factor that increases SCN1A transcription (eTFSCN1A), is being tested in a phase II clinical trial to treat Dravet syndrome, a severe epileptic encephalopathy caused by heterozygous loss of function of SCN1A. Finally, AAV-based downregulation of Gys1 through CRISPR-CAS9 or a miRNA AAV9 are being tested in animal models of adult polyglucosan body disease and Lafora disease. These two strategies result in a remarkable reduction of polyclucosan body formation and a slight reduction of some neuroinflammatory markers.

However, there is still an unmet medical need of finding reliable therapeutic strategies for the treatment of Lafora disease. The present invention is focused on solving this problem and a novel therapeutic strategy is herein provided.

### DESCRIPTION OF THE INVENTION

### Brief description of the invention

The present invention refers to a nucleic acid vector to be used in the context of gene therapy, in the treatment of Lafora disease. Particularly, the inventors of the present invention have designed a novel rAAV vector containing the human EPM2A gene, rAAV2/9-CAG-hEPM2A, which improves the neurological alterations present in the Epm2a-/- mouse model of Lafora disease by recovering the function of the Epm2a gene, preferably three months after a single intracerebroventricular (ICV) injection.

Although some AAV vectors have shown early healing effects within the clinic, some of them have raised doubts over their transduction performance and their tendency to set off an immune reaction in opposition to AAV transduced cells. In this regard, it is important to note that the vector of the invention (AAV2/9-CAG-hEPM2A ICV) shows a great capacity of transduction, especially in the hippocampus, and the transgene was correctly expressed throughout the life of mice.

Particularly, in Lafora disease, seizures are one of the most severe symptoms and may lead to increased morbidity and mortality. Of note, according to the results herein provided, the vector of the invention is able to prevent the appearance of myoclonic jerks after PTZ injections. In Epm2a-/- mice, AAV2/9-CAG-hEPM2A reduced hypersensitivity to PTZ. Moreover, it is herein shown that AAV2/9-CAG-hEPM2A prevents the onset of cognitive problems. On the other hand, treatment with AAV2/9-CAG-hEPM2A also resulted in a better outcome in other neurological features, such as spontaneous locomotor activity and motor coordination, lower amounts of LBs in the hippocampus, and reduced neuroinflammation and neurodegeneration.

In conclusion, AAV2/9-CAG-hEPM2A vector shows a clear efficacy, opening it possible clinical use for gene therapy purposes.

So, the first embodiment of the present invention refers to a nucleic acid vector (hereinafter "vector of the invention") comprising a coding sequence for EPM2A protein.

In a preferred embodiment of the invention, EPM2A protein is encoded by a nucleic acid sequence comprising the following sequence SEQ ID NO: 1:

ATGCGCTTCCGCTTTGGGGTGGTGGTGCCACCCGCCGTGGCCGGCGCCCGGCCGGAGCTGCTGGTGGGGT
CGCGGCCCGAGCTGGGGCGTTGGGAGCCGCGCGGTGCCGTCCGCCTGAGGCCGGCCGGCACCGCGGCGGGCG
ACGGGGCCCTGGCCCTGCAGGAGCCGGGCCTGTGGCTCGGGGAGGTGGAGCTGGCGGCCGAGGAGGCGGCGC
AGGACGGGGCGGAGCCGGGCCGCGTGGACACGTTCTGGTACAAGTTCCTGAAGCGGGAGCCGGGAGGAGAG
CTCTCCTGGGAAGGCAATGGACCTCATCATGACCGTTGCTGTACTTACAATGAAAACAACTTGGTGGATGGTG
TGTATTGTCTCCCAATAGGACACTGGATTGAGGCCACTGGGCACACCAATGAAATGAAGCACACAACAGACTT
CTATTTTAATATTGCAGGCCACCAAGCCATGCATTATTCAAGAATTCTACCAAATATCTGGCTGGGTAGCTGCC
CTCGTCAGGTGGAACATGTAACCATCAAACTGAAGCATGAATTGGGGATTACAGCTGTAATGAATTTCCAGAC
TGAATGGGATATTGTACAGAATTCCTCAGGCTGTAACCGCTACCCAGAGCCCATGACTCCAGACACTATGATT
AAACTATATAGGGAAGAAGGCTTGGCCTACATCTGGATGCCAACACCAGATATGAGCACCGAAGGCCGAGTA
CAGATGCTGCCCCAGGCGGTGTGCCTGCTGCATGCGCTGCTGGAGAAGGGACACATCGTGTACGTGCACTGCA
ACGCTGGGGTGGGCCGCTCCACCGCGGCTGTCTGCGGCTGGCTCCAGTATGTGATGGGCTGGAATCTGAGGAA
GGTGCAGTATTTCCTCATGGCCAAGAGGCCGGCTGTCTACATTGACGAAGAGGCCTTGGCCCGGGCACAAGAA
GATTTTTTCCAGAAATTTGGGAAGGTTCGTTCTTCTGTGTGTAGCCTGTAG

In a preferred embodiment of the invention, EPM2A protein comprises the following amino acid sequence SEQ ID NO: 2:

In a preferred embodiment of the invention, the vector of the invention is characterized by comprising the following SEQ ID NO: 3:

CAGCTGCGCGCTCGCTCGCTCACTGAGGCCGCCCGGGCAAAGCCCGGGCGTCGGGCGACCTTTGGTCGCCCGG
CCTCAGTGAGCGAGCGAGCGCGCAGAGAGGGAGTGGCCAACTCCATCACTAGGGGTTCCTTGTAGTTAATGAT
TAACCCGCCATGCTACTTATCTACTCGACATTGATTATTGACTAGTTATTAATAGTAATCAATTACGGGGTCAT
TAGTTCATAGCCCATATATGGAGTTCCGCGTTACATAACTTACGGTAAATGGCCCGCCTGGCTGACCGCCCAA
CGACCCCCGCCCATTGACGTCAATAATGACGTATGTTCCCATAGTAACGCCAATAGGGACTTTCCATTGACGT
CAATGGGTGGAGTATTTACGGTAAACTGCCCACTTGGCAGTACATCAAGTGTATCATATGCCAAGTACGCCCC
CTATTGACGTCAATGACGGTAAATGGCCCGCCTGGCATTATGCCCAGTACATGACCTTATGGGACTTTCCTACT
TGGCAGTACATCTACGTATTAGTCATCGCTATTACCATGGTCGAGGTGAGCCCCACGTTCTGCTTCACTCTCCC
CATCTCCCCCCCCTCCCCACCCCCAATTTTGTATTTATTTATTTTTTAATTATTTTGTGCAGCGATGGGGGCGGG
GGGGGGGGGGGGGCGCGCGCCAGGCGGGGCGGGGCGGGGCGAGGGGCGGGGCGGGGCGAGGCGGAGAGGT
GCGGCGGCAGCCAATCAGAGCGGCGCGCTCCGAAAGTTTCCTTTTATGGCGAGGCGGCGGCGGCGGCGGCCC
TATAAAAAGCGAAGCGCGCGGCGGGCGGGAGTCGCTGCGTTGCCTTCGCCCCGTGCCCCGCTCCGCGCCGCCT

CGCGCCGCCCGCCCCGGCTCTGACTGACCGCGTTACTCCCACAGGTGAGCGGGCGGGACGGCCCTTCTCCTCC
GGGCTGTAATTAGCGCTTGGTTTAATGACGGCTTGTTTCTTTTCTGTGGCTGCGTGAAAGCCTTGAGGGGCTCC
GGGAGGGCCCTTTGTGCGGGGGGAGCGGCTCGGGGGGTGCGTGCGTGTGTGTGTGCGTGGGGAGCGCCGCGT
GCGGCTCCGCGCTGCCCGGCGGCTGTGAGCGCTGCGGGCGCGGCGCGGGGCTTTGTGCGCTCCGCAGTGTGCG
CGAGGGGAGCGCGGCCGGGGGCGGTGCCCCGCGGTGCGGGGGGCTGCGAGGGGAACAAAGGCTGCGTGCGG
GGTGTGTGCGTGGGGGGGTGAGCAGGGGGTGTGGGCGCGTCGGTCGGGCTGCAACCCCCCCTGCACCCCCCTC
CCCGAGTTGCTGAGCACGGCCCGGCTTCGGGTGCGGGGCTCCGTACGGGGCGTGGCGCGGGGCTCGCCGTGCC
GGGCGGGGGGTGGCGGCAGGTGGGGGTGCCGGGCGGGGCGGGGCCGCCTCGGGCCGGGGAGGGCTCGGGGG
AGGGGCGCGGCGGCCCCCGGAGCGCCGGCGGCTGTCGAGGCGCGGCGAGCCGCAGCCATTGCCTTTTATGGT
AATCGTGCGAGAGGGCGCAGGGACTTCCTTTGTCCCAAATCTGTGCGGAGCCGAAATCTGGGAGGCGCCGCC
GCACCCCCTCTAGCGGGCGCGGGGCGAAGCGGTGCGGCGCCGGCAGGAAGGAAATGGGCGGGGAGGGCCTT
CGTGCGTCGCCGCGCCGCCGTCCCCTTCTCCCTCTCCAGCCTCGGGGCTGTCCGCGGGGGGACGGCTGCCTTCG
GGGGGGACGGGGCAGGGCGGGGTTCGGCTTCTGGCGTGTGACCGGCGGCTCTAGAGCCTCTGCTAACCATGTT
CATGCCTTCTTCTTTTTCCTACAGCTCCTGGGCAACGTGCTGGTTATTGTGCTGTCTCATCATTTTGGCAAAGAA
TTGATTAATTCGAGCGAACGCGTCGAGTCGCTCGGTACGATTTAAATTGAATTGGCCTCGAGCGCAAGCTTGA
GCTAGCATGCGCTTCCGCTTTGGGGTGGTGGTGCCACCCGCCGTGGCCGGCGCCCGGCCGGAGCTGCTGGTGG
TGGGGTCGCGGCCCGAGCTGGGGCGTTGGGAGCCGCGCGGTGCCGTCCGCCTGAGGCCGGCCGGCACCGCGG
CGGGCGACGGGGCCCTGGCCCTGCAGGAGCCGGGCCTGTGGCTCGGGGAGGTGGAGCTGGCGGCCGAGGAGG
CGGCGCAGGACGGGGCGGAGCCGGGCCGCGTGGACACGTTCTGGTACAAGTTCCTGAAGCGGGAGCCGGGAG
GAGAGCTCTCCTGGGAAGGCAATGGACCTCATCATGACCGTTGCTGTACTTACAATGAAAACAACTTGGTGGA
TGGTGTGTATTGTCTCCCAATAGGACACTGGATTGAGGCCACTGGGCACACCAATGAAATGAAGCACACAACA
GACTTCTATTTTAATATTGCAGGCCACCAAGCCATGCATTATTCAAGAATTCTACCAAATATCTGGCTGGGTAG
CTGCCCTCGTCAGGTGGAACATGTAACCATCAAACTGAAGCATGAATTGGGGATTACAGCTGTAATGAATTTC
CAGACTGAATGGGATATTGTACAGAATTCCTCAGGCTGTAACCGCTACCCAGAGCCCATGACTCCAGACACTA
TGATTAAACTATATAGGGAAGAAGGCTTGGCCTACATCTGGATGCCAACACCAGATATGAGCACCGAAGGCC
GAGTACAGATGCTGCCCCAGGCGGTGTGCCTGCTGCATGCGCTGCTGGAGAAGGGACACATCGTGTACGTGCA
CTGCAACGCTGGGGTGGGCCGCTCCACCGCGGCTGTCTGCGGCTGGCTCCAGTATGTGATGGGCTGGAATCTG
AGGAAGGTGCAGTATTTCCTCATGGCCAAGAGGCCGGCTGTCTACATTGACGAAGAGGCCTTGGCCCGGGCAC
AAGAAGATTTTTTCCAGAAATTTGGGAAGGTTCGTTCTTCTGTGTGTAGCCTGTAGGCGGCCGCAAGAATTCG
ATATCAAGCTTATCGATAATCAACCTCTGGATTACAAAATTTGTGAAAGATTGACTGGTATTCTTAACTATGTT
GCTCCTTTTACGCTATGTGGATACGCTGCTTTAATGCCTTTGTATCATGCTATTGCTTCCCGTATGGCTTTCATT
TTCTCCTCCTTGTATAAATCCTGGTTGCTGTCTCTTTATGAGGAGTTGTGGCCCGTTGTCAGGCAACGTGGCGT
GGTGTGCACTGTGTTTGCTGACGCAACCCCCACTGGTTGGGGCATTGCCACCACCTGTCAGCTCCTTTCCGGGA
CTTTCGCTTTCCCCCTCCCTATTGCCACGGCGGAACTCATCGCCGCCTGCCTTGCCCGCTGCTGGACAGGGGCT
CGGCTGTTGGGCACTGACAATTCCGTGGTGTTGTCGGGGAAATCATCGTCCTTTCCTTGGCTGCTCGCCTGTGT
TGCCACCTGGATTCTGCGCGGGACGTCCTTCTGCTACGTCCCTTCGGCCCTCAATCCAGCGGACCTTCCTTCCC
GCGGCCTGCTGCCGGCTCTGCGGCCTCTTCCGCGTCTTCGCCTTCGCCCTCAGACGAGTCGGATCTCCCTTTGG
GCCGCCTCCCCGCCTGATCGATACCGTCGACCTCGAGGGGGGGCCCGGTACCCGGGAATCAATTCACTCCTCA
GGTGCAGGCTGCCTATCAGAAGGTGGTGGCTGGTGTGGCCAATGCCCTGGCTCACAAATACCACTGAGATCTT
TTTCCCTCTGCCAAAAATTATGGGGACATCATGAAGCCCCTTGAGCATCTGACTTCTGGCTAATAAAGGAAAT
TTATTTTCATTGCAATAGTGTGTTGGAATTTTTTGTGTCTCTCACTCGGAAGGACATATGGGAGGGCAAATCAT
TTAAAACATCAGAATGAGTATTTGGTTTAGAGTTTGGCAACATATGCCCATATGCTGGCTGCCATGAACAAAG
GTTGGCTATAAAGAGGTCATCAGTATATGAAACAGCCCCCTGCTGTCCATTCCTTATTCCATAGAAAAGCCTTG
ACTTGAGGTTAGATTTTTTTTATATTTTGTTTTGTGTTATTTTTTTCTTTAACATCCCTAAAATTTTCCTTACATG
TTTTACTAGCCAGATTTTTCCTCCTCTCCTGACTACTCCCAGTCATAGCTGTCCCTCTTCTCTTATGGAGATCCC

TCGACCTGCAGCCCAAGCTGTAGATAAGTAGCATGGCGGGTTAATCATTAACTACAAGGAACCCCTAGTGATG
GAGTTGGCCACTCCCTCTCTGCGCGCTCGCTCGCTCACTGAGGCCGGGCGACCAAAGGTCGCCCGACGCCCGG
GCTTTGCCCGGGCGGCCTCAGTGAGCGAGCGAGCGCGCAGCTGGCGTAATAGCGAAGAGGCCCGCACCGATC
GCCCTTCCCAACAGTTGCGCAGCCTGAATGGCGAATGGCGATTCCGTTGCAATGGCTGGCGGTAATATTGTTC
TGGATATTACCAGCAAGGCCGATAGTTTGAGTTCTTCTACTCAGGCAAGTGATGTTATTACTAATCAAAGAAG
TATTGCGACAACGGTTAATTTGCGTGATGGACAGACTCTTTTACTCGGTGGCCTCACTGATTATAAAAACACTT
CTCAGGATTCTGGCGTACCGTTCCTGTCTAAAATCCCTTTAATCGGCCTCCTGTTTAGCTCCCGCTCTGATTCTA
ACGAGGAAAGCACGTTATACGTGCTCGTCAAAGCAACCATAGTACGCGCCCTGTAGCGGCGCATTAAGCGCG
GCGGGTGTGGTGGTTACGCGCAGCGTGACCGCTACACTTGCCAGCGCCCTAGCGCCCGCTCCTTTCGCTTTCTT
CCCTTCCTTTCTCGCCACGTTCGCCGGCTTTCCCCGTCAAGCTCTAAATCGGGGGCTCCCTTTAGGGTTCCGATT
TAGTGCTTTACGGCACCTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGTTCCAAACTGGAACAAC
ACTCAACCCTATCTCGGTCTATTCTTTTGATTTATAAGGGATTTTGCCGATTTCGGCCTATTGGTTAAAAAATG
AGCTGATTTAACAAAAATTTAACGCGAATTTTAACAAAATATTAACGCTTACAATTTAAATATTTGCTTATACA
ATCTTCCTGTTTTTGGGGCTTTTCTGATTATCAACCGGGGTACATATGATTGACATGCTAGTTTTACGATTACCG
TTCATCGATTCTCTTGTTTGCTCCAGACTCTCAGGCAATGACCTGATAGCCTTTGTAGAGACCTCTCAAAAATA
GCTACCCTCTCCGGCATGAATTTATCAGCTAGAACGGTTGAATATCATATTGATGGTGATTTGACTGTCTCCGG
CCTTTCTCACCCGTTTGAATCTTTACCTACACATTACTCAGGCATTGCATTTAAAATATATGAGGGTTCTAAAA
ATTTTTATCCTTGCGTTGAAATAAAGGCTTCTCCCGCAAAAGTATTACAGGGTCATAATGTTTTTGGTACAACC
GATTTAGCTTTATGCTCTGAGGCTTTATTGCTTAATTTTGCTAATTCTTTGCCTTGCCTGTATGATTTATTGGAT
GTTGGAATCGCCTGATGCGGTATTTTCTCCTTACGCATCTGTGCGGTATTTCACACCGCATATGGTGCACTCTC
AGTACAATCTGCTCTGATGCCGCATAGTTAAGCCAGCCCCGACACCCGCCAACACCCGCTGACGCGCCCTGAC
GGGCTTGTCTGCTCCCGGCATCCGCTTACAGACAAGCTGTGACCGTCTCCGGGAGCTGCATGTGTCAGAGGTT
TTCACCGTCATCACCGAAACGCGCGAGACGAAAGGGCCTCGTGATACGCCTATTTTTATAGGTTAATGTCATG
ATAATAATGGTTTCTTAGACGTCAGGTGGCACTTTTCGGGGAAATGTGCGCGGAACCCCTATTTGTTTATTTTT
CTAAATACATTCAAATATGTATCCGCTCATGAGACAATAACCCTGATAAATGCTTCAATAATATTGAAAAAGG
AAGAGTATGAGTATTCAACATTTCCGTGTCGCCCTTATTCCCTTTTTTGCGGCATTTTGCCTTCCTGTTTTTGCT
CACCCAGAAACGCTGGTGAAAGTAAAAGATGCTGAAGATCAGTTGGGTGCACGAGTGGGTTACATCGAACTG
GATCTCAACAGCGGTAAGATCCTTGAGAGTTTTCGCCCCGAAGAACGTTTTCCAATGATGAGCACTTTTAAAG
TTCTGCTATGTGGCGCGGTATTATCCCGTATTGACGCCGGGCAAGAGCAACTCGGTCGCCGCATACACTATTCT
CAGAATGACTTGGTTGAGTACTCACCAGTCACAGAAAAGCATCTTACGGATGGCATGACAGTAAGAGAATTAT
GCAGTGCTGCCATAACCATGAGTGATAACACTGCGGCCAACTTACTTCTGACAACGATCGGAGGACCGAAGG
AGCTAACCGCTTTTTTGCACAACATGGGGGATCATGTAACTCGCCTTGATCGTTGGGAACCGGAGCTGAATGA
AGCCATACCAAACGACGAGCGTGACACCACGATGCCTGTAGCAATGGCAACAACGTTGCGCAAACTATTAAC
TGGCGAACTACTTACTCTAGCTTCCCGGCAACAATTAATAGACTGGATGGAGGCGGATAAAGTTGCAGGACCA
CTTCTGCGCTCGGCCCTTCCGGCTGGCTGGTTTATTGCTGATAAATCTGGAGCCGGTGAGCGTGGGTCTCGCGG
TATCATTGCAGCACTGGGGCCAGATGGTAAGCCCTCCCGTATCGTAGTTATCTACACGACGGGGAGTCAGGCA
ACTATGGATGAACGAAATAGACAGATCGCTGAGATAGGTGCCTCACTGATTAAGCATTGGTAACTGTCAGACC
AAGTTTACTCATATATACTTTAGATTGATTTAAAACTTCATTTTTAATTTAAAAGGATCTAGGTGAAGATCCTT
TTTGATAATCTCATGACCAAAATCCCTTAACGTGAGTTTTCGTTCCACTGAGCGTCAGACCCCGTAGAAAAGAT
CAAAGGATCTTCTTGAGATCCTTTTTTTCTGCGCGTAATCTGCTGCTTGCAAACAAAAAAACCACCGCTACCAG
CGGTGGTTTGTTTGCCGGATCAAGAGCTACCAACTCTTTTTCCGAAGGTAACTGGCTTCAGCAGAGCGCAGAT
ACCAAATACTGTTCTTCTAGTGTAGCCGTAGTTAGGCCACCACTTCAAGAACTCTGTAGCACCGCCTACATACC
TCGCTCTGCTAATCCTGTTACCAGTGGCTGCTGCCAGTGGCGATAAGTCGTGTCTTACCGGGTTGGACTCAAGA
CGATAGTTACCGGATAAGGCGCAGCGGTCGGGCTGAACGGGGGGTTCGTGCACACAGCCCAGCTTGGAGCGA

ACGACCTACACCGAACTGAGATACCTACAGCGTGAGCTATGAGAAAGCGCCACGCTTCCCGAAGGGAGAAAG
GCGGACAGGTATCCGGTAAGCGGCAGGGTCGGAACAGGAGAGCGCACGAGGGAGCTTCCAGGGGGAAACGC
CTGGTATCTTTATAGTCCTGTCGGGTTTCGCCACCTCTGACTTGAGCGTCGATTTTTGTGATGCTCGTCAGGGG
GGCGGAGCCTATGGAAAAACGCCAGCAACGCGGCCTTTTTACGGTTCCTGGCCTTTTGCTGGCCTTTTGCTCAC
ATGTTCTTTCCTGCGTTATCCCCTGATTCTGTGGATAACCGTATTACCGCCTTTGAGTGAGCTGATACCGCTCGC
CGCAGCCGAACGACCGAGCGCAGCGAGTCAGTGAGCGAGGAAGCGGAAGAGCGCCCAATACGCAAACCGCC
TCTCCCCGCGCGTTGGCCGATTCATTAATGCAGCTGCGCGCTCGCTCGCTCACTGAGGCC

In a preferred embodiment of the invention, the nucleic acid sequence encoding EPM2A is operably linked to a regulatory sequence that drives the expression of the coding sequence.

In a preferred embodiment of the invention, the vector of the invention is a non-integrative vector.

In a preferred embodiment of the invention, the vector of the invention is an adeno-associated virus-based non-integrative vector.

In a preferred embodiment of the invention, the vector of the invention is an adeno-associated virus-based non-integrative vector derived from a serotype 2 or 9 adeno-associated virus.

In a preferred embodiment of the invention, the capsid of the adeno-associated virus-based vector is made of a capsid proteins of the serotype 9 adeno-associated virus, and the nucleic acid sequence contained in the capsid is flanked at both ends by internal terminal repeats corresponding to serotype 2 adeno-associated virus.

In a preferred embodiment of the invention, the vector of the invention comprises a regulatory sequence which is a constitutive promoter.

In a preferred embodiment of the invention, the vector of the invention comprises a regulatory sequence which is the promoter CAG.

The second embodiment of the present invention refers to the vector of the invention for use as a medicament, for use in the treatment of a disease or medical condition caused by the following deletions: Epm2a-/- or Epm2b-/-, such as Lafora disease. Alternatively, the present invention refers to a method for the treatment of a disease or medical condition caused by the following deletions: Epm2a-/- or Epm2b-/-, such as Lafora disease, which comprises the administration of a therapeutically effective dose or amount of the vector of the invention, optionally in combination with pharmaceutically acceptable excipients or carriers.

In a preferred embodiment the route of administration may be selected from: intracerebroventricular, intrathecal or intravenous administration.

For the purpose of the present invention the following terms are defined:
- "Pharmaceutically acceptable excipient or carrier" refers to an excipient that may optionally be included along with the vector of the invention and that causes no significant adverse toxicological effects to the patient.
- By "therapeutically effective dose or amount" of the vector of the invention, the present invention refers to the situation when the vector of the invention is administered and brings about a positive therapeutic response in a subject. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.

### Brief description of the figures

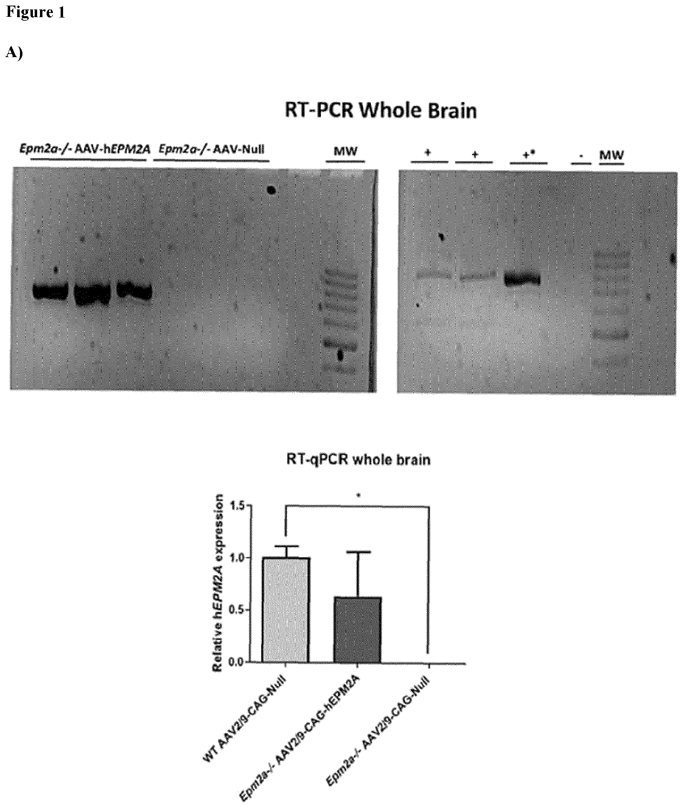

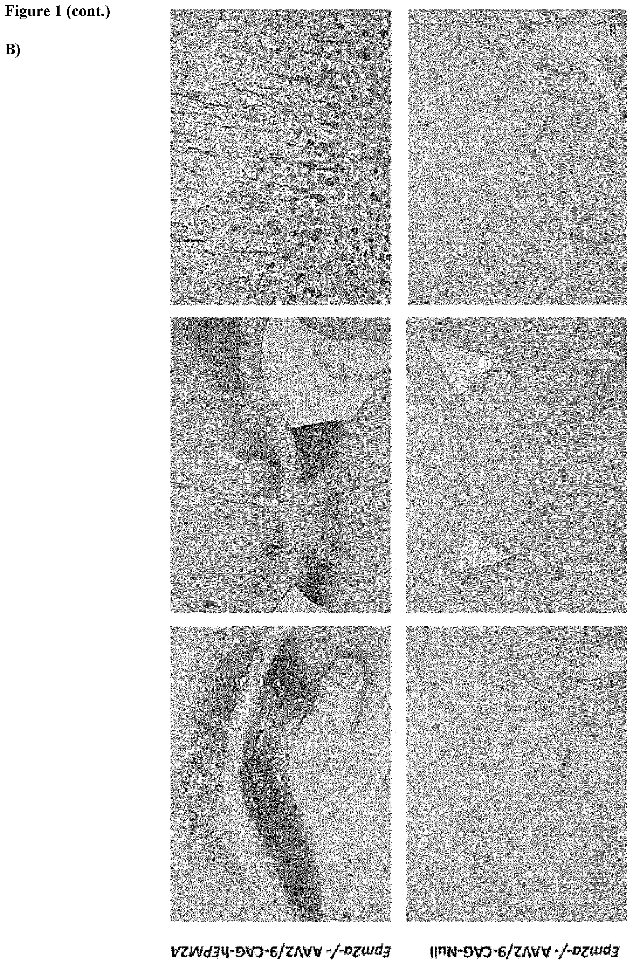

**Figure 1****. Transgene expression in treated *Epm2a*^{*-*/*-*} mice and vector transduction efficiency. (A)** Detection and relative quantification of Homo sapiens EPM2A glucan phosphatase laforin (*EPM2A*), transcript variant 1 mRNA from whole brain. *Epm2a-*/*-* AAV-*hEPM2A:* cDNA from mice injected with AAV2/9-CAG-h*EPM2A*; *Epm2a-*/*-* AAV-Null: cDNA from mice injected with AAV2/9-CAG-Null; + : cDNA Human sample; +* : cDNA cortex from treated mice; - : No sample; MW: Molecular Weight. **(B)** Laforin immunohistochemistry of *Epm2a-*/*-* treated and non-treated mice 3 months after injection. AAV2/9-CAG-h*EPM2A* efficiently transduces hippocampus, cortex and lateral ventricles.

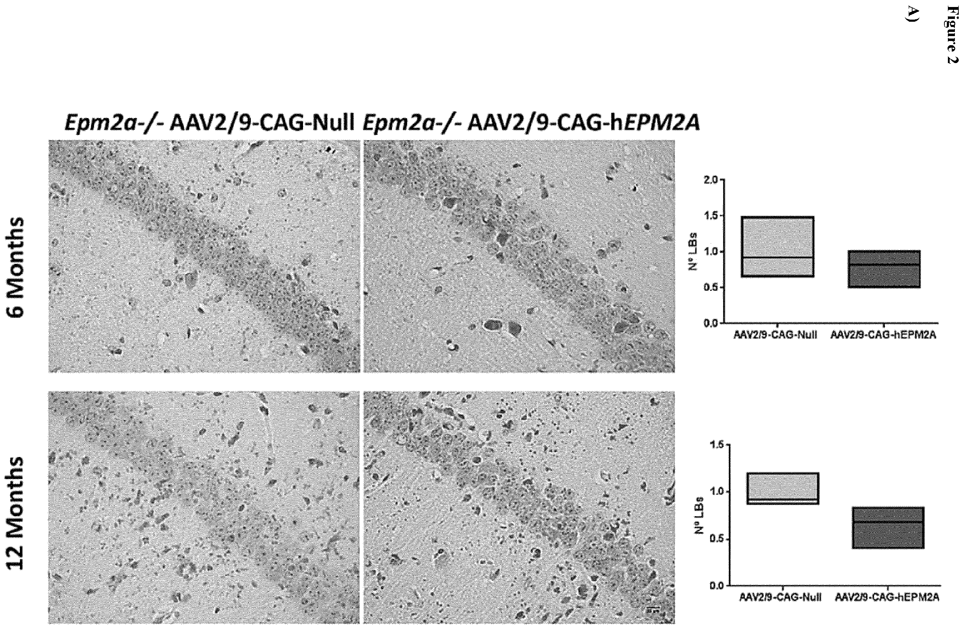

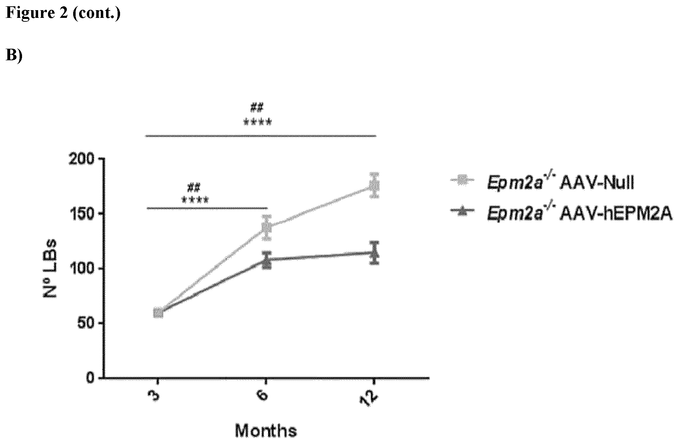

**Figure 2****. LB formation in treated vs non-treated mice. (A)** Quantitative comparison of LBs in the hippocampal CA1 region of treated and non-treated mice at 6 and 12 months of age (3- and 9-months post-injection). Data are shown as line at median of means. Means were normalized using non-treated *Epm2a-*/*-* mice values. A Mann-Whitney non-parametric test was carried out. A tendency to diminish the number of LBs in the hippocampus of Epm2a^{-/-}treated mice was observed, although no significance differences were found **(B)** Progression of LB formation from *Epm2a-*/*-*mice at 3 months of age (pre-injection) to treated and non-treated *Epm2a-*/*-* mice at 6 and 12 months of age (3- and 9-months post-injection). Data are shown as a mean ± SEM. A two-way ANOVA test was performed. * p<0.05, ** p<0.01, **** p<0.0001: * for LB progression in *Epm*2*a* -/- AAV2/9-CAG-Null; # for LB progression in *Epm*2*a* -/- AAV2/9-CAG-hEPM2A.

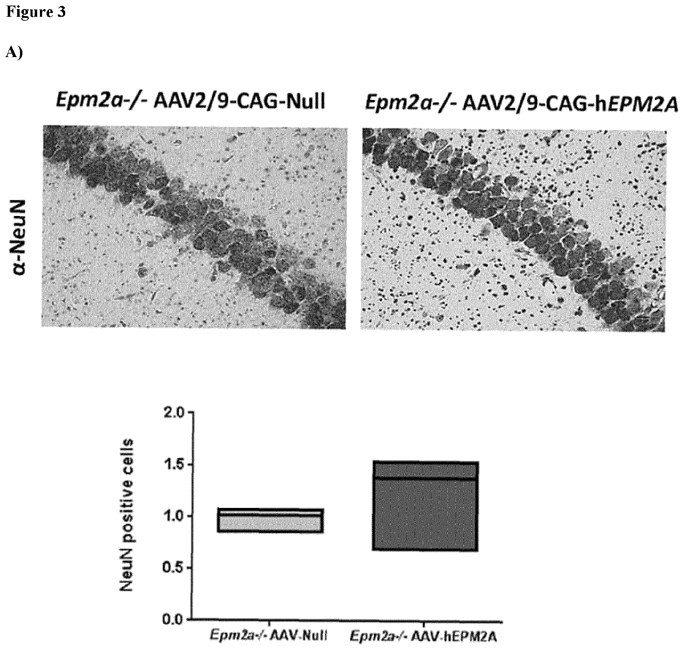

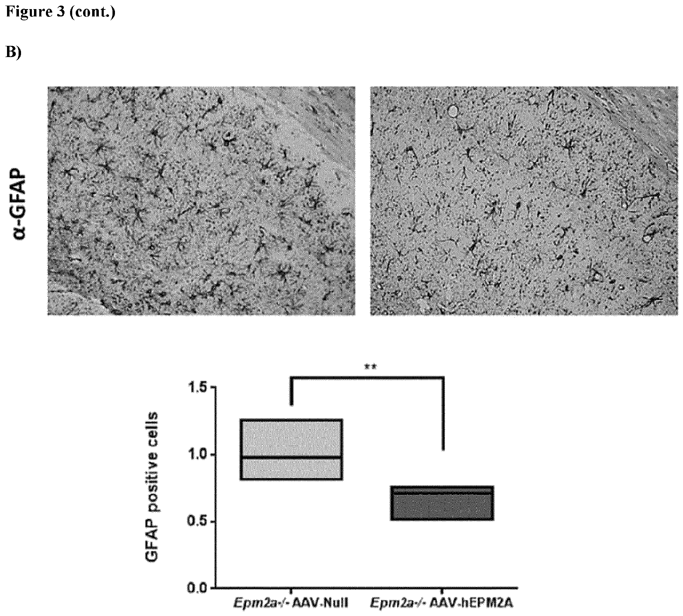

**Figure 3****. Neurodegeneration and neuroinflammation in treated vs non-treated *Epm2a-*/*-* mice. (A)** NeuN immunostaining and quantification of positive CA1 hippocampal neurons from brain samples of *Epm2a-*/*-* treated and non-treated mice 3 months after injection. Data are shown as line at median of means. Means were normalized to values from *Epm2a-*/*-* non-treated mice. A non-parametric Mann-Whitney test was performed. No significance differences were observed. **(B)** GFAP immunostaining and quantification of positive CA1 hippocampal reactive astrocytes from brain samples of *Epm2a*^{-/-} treated and non-treated mice 3 months after injection. Data are shown as line at median of means. Means were normalized to values from *Epm2a*^{*-*/*-*} non-treated mice. A non-parametric Mann-Whitney test was carried out. *p<0.05; **p<0.01.

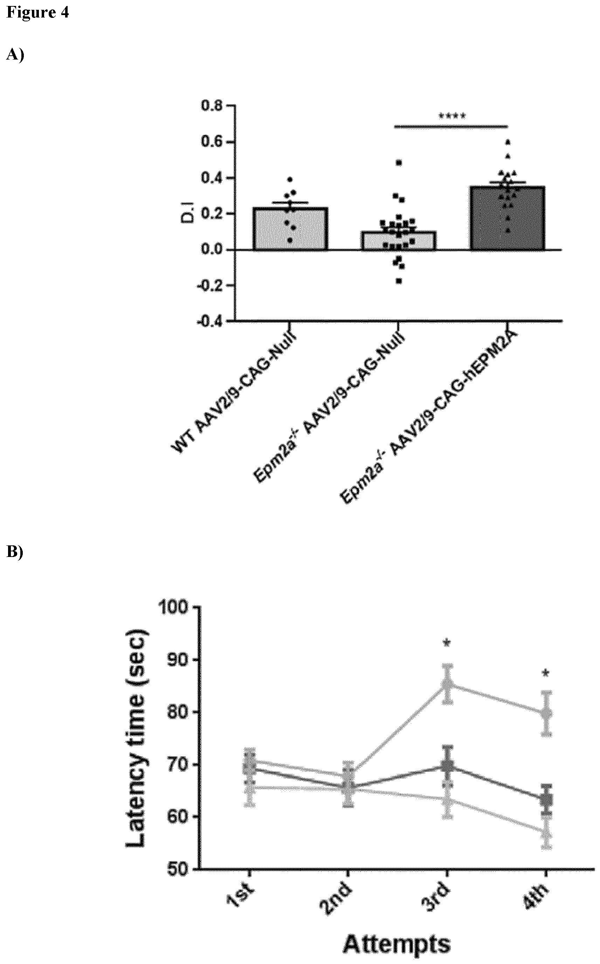

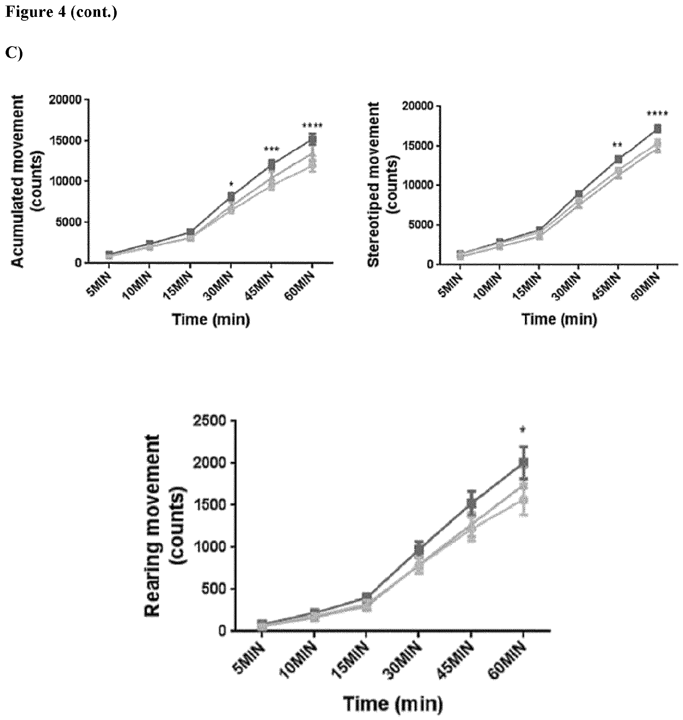

**Figure 4****. Cognitive and motor behaviour of *Epm2a-*/*-* treated and non-treated mice. (A)** Evaluation of sporadic memory with the ORT in WT and *Epm2a*^{*-*/*-*} treated and non-treated mice 3 months after ICV injection. Data are shown as the D.I mean ± SEM of each condition. A one-way ANOVA test was carried out with Turkey's multiple comparisons between the experimental groups. * p<0.05, ** p<0.01, *** p<0.001, **** p<0.0001. **(B)** Latency time to fall from the rotarod cylinder in WT and *Epm2a*^{-/-} treated and non-treated mice 3 months after ICV injection. Data are shown as the latency mean ± SEM of each attempt in each condition. A one-way ANOVA test was carried out with Turkey's multiple comparisons between the experimental groups. * p<0.05, ** p<0.01, *** p<0.001, **** **(C)** Analysis with the SEDACOM 1.4 software of accumulated, stereotyped and rearing movement of WT and *Epm2a*^{*-*/*-*} treated and non-treated mice 3 months after ICV injection. Data are shown as a mean ± SEM of the counts of each condition. A two-way ANOVA test was performed. * p<0.05, ** p<0.01, **** p<0.0001.

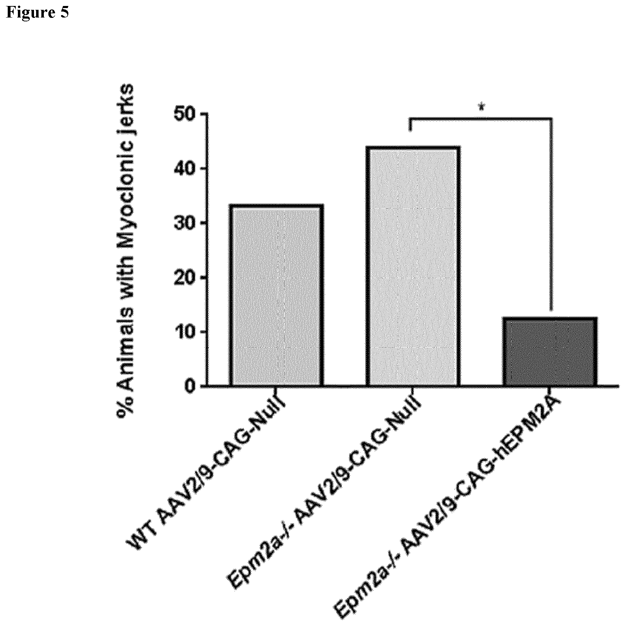

**Figure 5****. Sensitivity to PTZ at 30 mg/kg.** Percentage of mice with myoclonic jerks after intraperitoneal injection of PTZ at 30 mg/kg in WT and *Epm2a*^{*-*/*-*} treated and non-treated mice 3 months after ICV injection. Data are shown as the percentage of mice with myoclonic jerks. A one-way ANOVA test was performed with Turkey's multiple comparisons between the experimental groups. * p<0.05, ** p<0.01, *** p<0.001, **** p<0.0001.

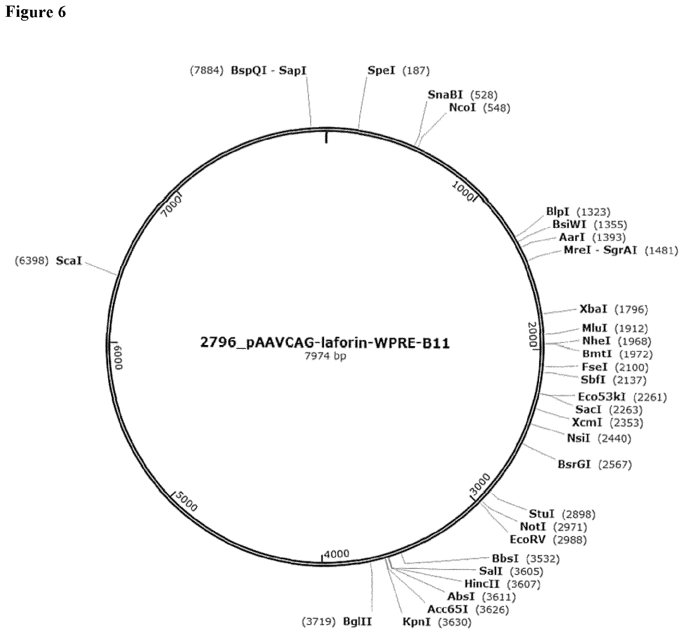

**Figure 6****. Representation of the vector of the invention.**

### EXAMPLES

The present invention is illustrated by means of the Examples set below, without the intention of limiting its scope of protection.

### Example 1. MATERIAL AND METHODS

### Example 1.1. Experimental Animals

The *Epm2a*^{*-*/*-*} mouse model of Lafora disease was generated as previously described *[*Ganesh S, Delgado-Escueta AV, Sakamoto T, Avila MR, Machado-Salas J, Hoshii Y, et al. Targeted disruption of the Epm2a gene causes formation of Lafora inclusion bodies, neurodegeneration, ataxia, myoclonus epilepsy and impaired behavioral response in mice. Hum Mol Genet. 2002; 11(11):1251-62] and age-matched wild-type animals (C57BL6) were used as controls. The mouse colonies were bred in the Animal Facility Service of the Instituto de Investigation Sanitaria (IIS)-Fundación Jiménez Diaz, housed in isolated cages with a 12:12 light/dark cycle under constant temperature (23 °C), with free access to food and water. All the experiments were carried out using and sacrificing the minimum number of animals and minimizing their suffering. Experiments were conducted in accordance with the "Principles of Laboratory Animal Care" (NIH publication No. 86-23, revised 1985), as well as with the European Communities Council Directive (2010/63/EU) and the Ethical Review Board of the Instituto de Investigation Sanitaria-Fundacion Jimenez Diaz.

### Example 1.2. Production of rAAV2/9-CAG-hEPM2A and rAAV2/9-CAG-hEPM2B vectors

The rAAV2/9-CAG-hEPM2A, containing the cDNA of the *EPM2A* gene transcript variant 1 (hE*PM2A)* (NM_005670.4), and the rAAV2/9-CAG-NULL, containing an empty vector, were generated in the Unitat de Producci6 de Vectors (UPV_ www.viralvector.eu) following the triple transfection system: (a) the ITR-containing plasmid; (b) the plasmid encoding AAV capsid (VP1, VP2 and VP3 proteins) and replicate genes; and (c) the adenoviral helper plasmid. To remove empty capsids, AAV vectors were purified by iodixanol-based ultracentrifugation.

### Example 1.3. Stereotactic intracerebroventricular (ICV) injections

*Epm2a*^{*-*/*-*} mice were treated at 3 months of age with ICV injections of AAV2/9-CAG-hE*PM2A* or AAV2/9-CAG-Null vectos. Animals were deeply anesthetized in an induction chamber filled with 4% isoflurane and 2% O₂ and maintained with 2% isoflurane and 1.5% O₂. Mice were fixed in a stereotaxic frame (Stoelting, Illinois, USA). Body temperature was regulated placing the animals on the heating pad at 37°C. Hydration was controlled by subcutaneous injection of saline (1 mL) and ocular dryness was avoided by smearing ophthalmic gel. The incision site was sterilized with 70% ethanol, and a 2 cm incision was made in the midline, starting behind the eyes. H₂O₂ was applied to the skull to show bregma and lambda. A small burr hole was drilled according to the stereotaxic coordinates of the right cerebral lateral ventricle relative to bregma (anterioposterior -0.3 and mediolateral -0.9). A Hamilton^{®} syringe (ThermoFisher Scientific, Massachusetts, USA, Cat. #10664301) was introduced at -2.5 cm in the dorsoventral axis to deliver 3 µL of viral suspension with a viral title of 1.26 × 10¹² vg/mL at a rate of 3 µL/min. The incision was sutured, and Meloxicam (Boehringer Ingelheim, Georgia, USA) (5mg/kg) was administrated as an analgesic.

### Example 1.4. Spontaneous locomotor activity

Accumulated spontaneous movements were monitored with a computerized actimeter (Harvard Apparatus, Holliston, MA, USA). The device registers the number of times each mouse crosses the open field by measuring the number of infrared light beam breaks, and the SEDACOM 1.4 software (Harvard Apparatus, Holliston, MA, USA) analyzed the spontaneous, rearing, and stereotyped movements after 5, 10, 15, 30, 45, and 60 min.

### Example 1.5. Motor coordination

The rotarod test (Harvard Apparatus, Holliston, MA, USA) was used to measure motor coordination and balance. Mice were trained for 2 consecutive days. On day 1, mice were placed on the rotarod for 60 s at a constant speed (4 rpm). On day 2, they were placed on the rotarod at 4 rpm and trained to stay on the cylinder at increasing speed (from 4 to 8 rpm). On the following 2 days, to minimize differences due to learning difficulties, only mice that were able to stay on the rod for 60 s were analyzed in the test sessions. The latency time to fall down from the cylinder at an increasing speed from 4 to 40 rpm was recorded in two sessions per day, for a maximum period of 5 min.

### Example 1.6. Object recognition task (ORT)

This test was applied to study episodic memory retention. Mice were individually habituated to an open field dark box for 10 min. After 2 h, two identical objects (A and B) were placed in the center of the box. Each isolated mouse freely explored objects A and B, and the examination of both objects (tA and tB) was measured. The test was performed 2 h later. A new object C was placed in the box instead of object B, and the exploration time of each object (tA and tC) was recorded. Exploration times were measured using several virtual timers generated by the XNote Stopwatch software and activated while the mice were inspecting the object from 2 cm or less. A discrimination index (D.I.) was calculated following this equation: D.I. = (tC-tA) / (tC + tA).

### Example 1.7. Anxiety test

Actitrack 2.7.13 software (PanLab/Harvard apparatus) allowed registering the percentage of time that each mouse spent in the center zone of the open field box for 10 minutes compared to the percentage of time each mouse spent in the surrounding area.

### Example 1.8. Sensitivity to Pentylenetetrazole (PTZ)

PTZ (Merck, Darmstadt, Germany) was used to analyze neuronal hyperexcitability in mice. PTZ was administered intraperitoneally in a single injection. Two different doses were used: 30 mg/kg (subconvulsive dose, hardly produces GTC seizures in WT animals) and 50 mg/kg (convulsive dose). The subconvulsive dose was used to assess the percentage of mice that had myoclonic jerks. The convulsive dose, which has been described to induce GTC seizures in 50% of WT animals, was administered to evaluate the percentage of animals with GTC seizures, their length, and the latency to first myoclonic or GTC seizure. No animal presented more than a single GTC seizure after each injection. The lethality due to the administration of this dose was also calculated. Each animal was examined for 45 min by two different researchers.

### Example 1.9. RNA extraction and quantitative reverse transcription-polymerase chain reaction (RT-qPCR)

RNA was extracted from brain samples previously homogenized on ice with TRIzol^{™} Reagent (ThermoFisher Scientific, Massachusetts, US). RNA pellets were washed, dried, resuspended, treated with DNase Enzyme (ThermoFisher Scientfic, Massachusetts, US) and quantified with a NanoDrop ND-1000 spectrophotometer (ThermoFisher, Massachusetts, US). RT-PCR experiments were carried out using the High-Capacity cDNA Reverse Transcription Kit with RNase inhibitor (ThermoFisher Scientific, Massachusetts, US) and 1 µg RNA per reaction. The RT-PCR conditions were: 25°C for 10 min - 37°C for 120 min - 5 min for 85°C. *hEPM2A* transcript variant 1 cDNA was used as a template for RT-qPCR. The reaction was carried out using TaqMan^{™} Fast Advanced Master Mix (ThermoFisher Scientific, MA, USA), with *Epm2a, EPM2A* and *Gapdh* probes (ThermoFisher Scientific, MA, USA). The qPCR conditions were: 50°C for 2 min - 95°C for 2 min - 95°C for 1 sec + 60°C for 20 sec (40 cycles). The analysis was performed with the 2^{-ΔΔCT} method.

### Example 1.10. Immunohistochemistry assays and PAS staining

Mice were anesthetized and transcardially perfused with 4% phosphate-buffered paraformaldehyde. Brains were removed, dehydrated, and embedded in paraffin. The blocks were sectioned into consecutive 5-µm-thick sections. For PAS-diastase (PAS-D) staining, sections were rehydrated in decreasing graded alcohols, processed with porcine pancreas α-amylase (5 mg/ml in dH2O) (Merck, Darmstadt, Germany) and the PAS Kit (Merck, Darmstadt, Germany), and counterstained with Gill No. 3 hematoxylin (Merck, Darmstadt, Germany). For immunohistochemistry, rehydrated sections were incubated in boiling 0.1 M sodium citrate buffer, pH 6.0, for antigen retrieval. Samples were incubated with blocking buffer (1% bovine serum albumin, 5% fetal bovine serum, 2% Triton X-100, diluted in PBS) and with primary antibodies diluted in blocking buffer. The primary antibodies used were neuronal nuclei (NeuN) antibody (Cat. # MAB377) (1:100 dilution) (Millipore, Temecula, CA, USA), glial fibrillary acidic protein (GFAP) antibody (Cat. # MAB360) (1:1000 dilution) (Millipore, Temecula, CA, USA) and laforin antibody (3µg/mL) (Lifespan Biosciences, Washington, US, Cat. #LS-B6474). Subsequently, sections were stained with the Vectastain ABC kit (Vector Laboratories, Burlingame, CA, USA). Immunoreactivity was developed using diaminobenzidine (Dako Cytomation, CA, USA) and H₂O₂. The sections were counterstained with Carazzi hematoxylin (Panreac Quimica, Barcelona, Spain). For histological assays, samples from 4 mice per experimental group were used.

Two consecutive sections per animal were stained and analyzed to ensure reproducibility. Images from the same area of the CA1 region of each hippocampus were acquired with a Leica DMLB 2 (Leica, Wetzlar, Germany), connected to a Leica DFC320 FireWire digital microscope camera (Leica, Wetzlar, Germany). Finally, LBs and NeuN- or GFAP-positive cells were quantified by two different researchers using ImageJ software (NIH, Bethesda, MD, USA). Each value represented is the mean of those quantifications.

### Example 1.11. Statistical analysis

Values are given as mean ± standard error of mean (SEM) or as percentages. Differences between experimental groups were analyzed by one- or two-way ANOVA, Fisher's exact, non-parametric Kruskal-Wallis, or Mann-Whitney tests, as indicated in each case. Statistical results were obtained using GraphPad Prism 6.0 (San Diego, CA). Statistical significance thresholds were *p < 0.05, **p < 0.01, ***p < 0.001, and ****p < 0.0001.

### Example 1.12. Data Availability

Data supporting the findings of this study are available from the corresponding author, upon reasonable request.

### EXAMPLE 2. RESULTS

### Example 2.1. The transgene delivered by the rAAV2/9-CAG-hEPM2A vector is correctly transcribed and translated, and the vector is transduced throughout the central nervous system (CNS)

We verified and quantified transcription of the transgene by RT-PCR and RT-qPCR **(****Figure 1A****).** *hEPM2A* transcript variant 1 cDNA obtained from whole brain mRNA was used as a template for RT-qPCR. In Epm2a-/- mice, expression of the *hEPM2A* transcript was 60% of that observed in WT animals **(****Figure 1A****).** Transduction of the vector and translation of RNA were analyzed by immunohistochemistry with an anti-laforin antibody **(****Figure 1B****).** The protein was successfully expressed, and the vector was broadly spread throughout hippocampus, cortex and lateral ventricles **(****Figure 1B****).**

### Example 2.2. rAAV2/9-CAG-hEPM2A prevents LB formation in the brain of Epm2a^{-/-}treated mice

We analyzed the number of LBs in the CA1 region of the hippocampus of Epm2a-/- mice using PAS-D staining before rAAV2/9-CAG-h*EPM2A* injection at 3 months of age, and 3 and 9 months after rAAV2/9-CAG-hEPM2A ICV injection **(****Figure 2A****).** We observed that brains from mice treated with rAAV2/9-CAG-h*EPM2A* showed a lower progression of LB formation in the CA1 region compared to animals treated with the rAAV2/9-CAG-null vector **(****Figure 2B****).**

### Example 2.3. Injection of rAAV2/9-CAG-hEPM2A reduces neuronal loss and decreases astrogliosis in Epm2a^{-/-} treated mice

We also evaluated the effect of rAAV2/9-CAG-h*EPM2A* on neurodegeneration and neuroinflammation in *Epm2a*^{*-*/*-*} mice 3 months after injection using NeuN and GFAP antibodies. After quantifying the number of NeuN- and GFAP- positive cells present in the CA1 of *Epm2a*^{*-*/*-*} mice, we observed that the brains from treated mice showed a tendency to present higher numbers of NeuN-positive cells **(****Figure 3A****)** and fewer GFAP-immunostained cells than non-treated mice **(****Figure 3B****).**

### Example 2.4. rAAV2/9-CAG-hEPM2A vector diminishes memory and motor impairments in Epm2a-/- mice

We analyzed the effects of rAAV2/9-CAG-hEPM2A on episodic memory, motor coordination and spontaneous locomotor activity 3 months after injection. *Epm2a*^{*-*/*-*} treated mice showed a significant improvement in memory performance, with a higher discrimination index **(****Figure 4A****).** Treatment of Epm2a-/- mice with rAAV2/9-CAG-*hEPM2A* also improved motor coordination, showing an increased time to fall from the rod during the rotarod test **(****Figure 4B****).** In addition, *Epm2a*^{*-*/*-*} treated mice improved in spontaneous motor performance **(****Figure 4C****).**

### Example 2.5. rAAV2/9-CAG-hEPM2A vector decreases hypersensitivity to PTZ in Epm2a^{-/-} treated mice

We evaluated the benefits of rAAV2/9-CAG-h*EPM2A* on PTZ hypersensitivity in 6-month-old *Epm2a*^{*-*/*-*} mice, 3 months after injection. A subconvulsive dose of PTZ (30 mg/kg) resulted in a lower percentage of mice with myoclonic jerks **(****Figure 5****).**

## Claims

1. A nucleic acid vector comprising a coding sequence for EPM2A protein.

2. Nucleic acid vector, according to claim 1, wherein EPM2A protein is encoded by a nucleic acid sequence comprising the sequence SEQ ID NO: 1.

3. Nucleic acid vector, according to any of the previous claims, wherein EPM2A protein comprises the amino acid sequence SEQ ID NO: 2.

4. Nucleic acid vector, according to any of the previous claims, **characterized by** comprising the SEQ ID NO: 3.

5. Nucleic acid vector, according to any of the previous claims, wherein the nucleic acid sequence encoding EPM2A is operably linked to a regulatory sequence that drives the expression of the coding sequence.

6. Nucleic acid vector, according to any of the previous claims, wherein the vector is a non-integrative vector.

7. Nucleic acid vector, according to any of the previous claims, wherein the vector is an adeno-associated virus-based non-integrative vector.

8. Nucleic acid vector, according to any of the previous claims, wherein the vector is an adeno-associated virus-based non-integrative vector derived from a serotype 2 or 9 adeno-associated virus.

9. Nucleic acid vector, according to any of the previous claims, wherein the capsid of the adeno-associated virus-based vector is made of a capsid proteins of the serotype 9 adeno-associated virus, and the nucleic acid sequence contained in the capsid is flanked at both ends by internal terminal repeats corresponding to serotype 2 adeno-associated virus.

10. Nucleic acid vector, according to any of the previous claims, wherein the vector comprises a regulatory sequence which is a constitutive promoter.

11. Nucleic acid vector, according to any of the previous claims, wherein the vector comprises a regulatory sequence which is the promoter CAG.

12. Nucleic acid vector, according to any of the previous claims, for use a as medicament.

13. Nucleic acid vector, according to any of the previous claims, for use in the treatment of Lafora disease.
